# EUROPEAN PATENT APPLICATION

(11) **EP 2 749 640 A1**
(43) Date of publication of application: **02.07.2014**
(21) Application number: 12008615.2
(22) Date of filing: 27.12.2012
(51) Int. Cl.: C12N 5/0789, C12N 5/071

(54) **Processes for Producing Ectodermal, Mesodermal and Endodermal Cells as well as Pluripotent Stem Cells, Haematopoietic Stem Cells, Side Population Cells and Mesenchymal Stem Cells, and a Method of De-differentiating Peripheral Blood Cells**

(71) Applicant: Becker-Kojic, Zorica, 69181 Leimen (DE)
(72) Inventor: Becker-Kojic, Zorica, 69181 Leimen (DE)

(57) **Abstract**

The present invention relates to processes for producing ectodermal, mesodermal and endodermal cells as well as pluripotent stem cells, haematopoietic stem cells, side population cells and mesenchymal stem cells, comprising the step of activating the surface protein ACA. The present invention further relates to a method of de-differentiating peripheral blood cells by activating the surface protein ACA and to activators of the surface protein ACA for use in regenerative medicine and/or transplantation medicine.

## Description

The present invention relates to processes for producing ectodermal, mesodermal and endodermal cells as well as pluripotent stem cells, haematopoietic stem cells, side population cells and mesenchymal stem cells. The present invention further relates to a method of de-differentiating peripheral blood cells and to activators of the surface protein ACA.

Stem cells are biological cells found in all multicellular organisms that can be defined by their function to self-renew and produce more stem cells, and to differentiate into multiple specialized cell type. There are two main group of stem cells: adult stem cells that can be found in various tissues acting as a repair system for adult tissue, and pluripotent, such as embryonic stem cells, which are isolated from the inner cell mass of blastocysts and can differentiate into all the specialized cells, but also maintain the normal turnover of regenerative organs, such as blood, skin, or intestinal tissues.

Haematopoietic stem cells (HSC) are rare adult stem cells in the body that give rise to all lineages of the blood. These cells must balance the processes of self-renewal and differentiation in order to provide sufficient stem cells to sustain haematopoiesis and to generate more differentiated progeny. Stem cell maintenance and cell fate decisions of the haematopoietic system are tightly controlled by a few highly conserved developmentally regulated signalling pathways in order to assure a persistent pool of regenerating cells without overgrowth of immature cell type.

Side population cells are rare primitive cells with ability to efflux fluorescent DNA-binding dye Hoechst 33342 by membrane efflux pumps of the ATP-binding cassette (ABC) transporter superfamily. These cells represent 0.03% - 0.09% viable cells in human BM (Goodell MA et al., 1997, Nat Med 3, 1337-45). SP cells are typically negative for most lineage markers and CD34 antigen, and are highly enriched for long-term repopulating cells with extensive lymphoid and myeloid repopulating ability in sublethally irradiated, non-obese, diabetic/severe, combined immunodeficient mice (NOD-SCID).

Mesenchymal stem cells (MSCs) are multipotent stromal cells that can differentiate into various cell types including bone, cartilage and fat cells (Phinney DG et al., 2007, Stem Cells 25, 11; 2896-902).

It is a matter of fact that bone marrow (BM) and HSC transplants therapies have brought about permanent cures for numerous patients suffering from blood disorders. Unfortunately, for other patients there remain a 10-50% rate of transplanted mortalities and only a small chance of finding a suitable donor. Thus the transplantation medicine seems far away from offering an optimum cure for the majority of patients with blood disorder.

Sources of stem cells for clinical transplantation for treating and curing leukaemia, autoimmune diseases and other blood disorders, include bone marrow (BM), umbilical cord blood (UCB) or cytokine-mobilized peripheral blood (PB). Successful immune reconstitution with HSC is highly dependent on the number of CD34+ cells transplanted/kg body weight. However, BM and especially single UCB extraction provide insufficient numbers of CD34+ cells for adult transplantation, often requiring the pooling of multiple cord samples to obtain the optimal cell dose for full reconstitution. Complications related to the use of pooled samples include the possibility of graft-versus-graft or multiple graft-versus-host reactions and unpredictable reconstitution of each sample. Whereas highly purified HSC from half-matched donors can reconstitute a functional BM and nearly all cellular components of the blood in a marrow ablated patient, they failed to provide the lymphocytes necessary for rapid and complete immune reconstitution, such as T-cells. Thus the patient remains unprotected against potentially lethal viruses and other infectious agents and has a high risk of dying from infections. Attempts to overcome this problem by combining stem cell transplantation with infusions of unmanipulated lymphocytes from the donors have been prevented by the tendency of donor immune cells to react against the patient's organ and tissues causing graft-versus-host disease (GvHD). Such reactions are often lethal because of high degrees of mismatch between the donors and patient.

Leukapheresis is the method routinely used to obtain HSC. CD34+ cells are mobilized using growth factors into the peripheral blood and are collected for transplantation. With the help of different cocktails of growth factors, these cells could be amplified ex vivo. However, this method has several disadvantages. For the amplification of CD34 positive cells by cytokines, cell selection is needed from an initial cell concentration of around 10⁴ cells. Regulatory agents used are early acting and late acting cytokine cocktails which should be added every third day into culture in order to maintain a continuous presence of active cytokines avoiding apoptosis by deprivation. In most cases, combinations of growth factors that can induce potent proliferation cannot prevent the differentiation of HSC in long-term cultures and thus increase the possibility of GvHD.

Larger number of HSCs can also be generated by forced expression of cell intrinsic regulators, but perturbations resulting in unchecked self-renewal caused by gene-technical manipulation, does not include the control of overgrowth of immature cells that might participate in leukemogenesis.

Most culture conditions require a large starting number of CD34+ cells, and nonetheless result in net HSCs loses indicating that differentiation is favored over expansion. Thus, the possibility to maintain and expand long-term multilineage (including T-cells) reconstitution of HSC despite all factors and culture conditions available has not been achieved.

Adult stem cells have only a limited differentiation potential and cannot cross the lineage boundaries and differentiate into the cells of other germ layers, i.e. they are not pluripotent. Human embryonic stem cells (hESC) lines, autologous embryonic stem cells generated by therapeutic cloning and induced pluripotent stem cells (iPS) are the candidates for future applications in regenerative medicine. However, besides the ethical concerns of embryonic stem cell therapy, there is the immunological problem of graft-versus-host disease associated with allogeneic stem cell transplantation.

The induced pluripotent stem cells (iPSCs) are usually created by genetically modifying cells to overexpress four embryonic genes (Takahashi, K. et al., 2007, Cell 131, 861-872). The original protocol is based on retroviral mediated introduction of various transcription factors *Oct4, Sox2, Klf4,* and *c-Myc* (OSKC) or through other factor combinations that make them revert to an immature embryonic state.

The major drawback of using iPSCs is the limited understanding of molecular events underlying the reprogramming mechanism, which occur via many pathways and do not always lead to pluripotency. Reprogramming is slow, inefficient and a stochastic process and often incomplete. The resulting cells and their progeny bear a tumorigenic risk with potential genetic damage caused by using genetic manipulation, even with improvements in the virus-free and transgene-free reprogramming.

Another unresolved problem for ESC and iPS is teratoma, an indispensable criterion for pluripotency, but at the same time represents the main obstacle because of cancer potentially arisen from the residual number of undifferentiated pluripotent stem cells, thereby preventing their clinical application.

The present invention aims at overcoming the above described problems and drawbacks. Thus, the object of the present invention is to provide processes for producing ectodermal, mesodermal and endodermal cells as well as pluripotent stem cells, haematopoietic stem cells, side population cells and mesenchymal stem cells wherein these cells are produced in an ethically unobjectionable manner and in a sufficiently high number suitable for use in regenerative and transplantation medicine without bearing a tumorigenic risk or suffering from immunological problems.

A human GPI-linked glycoprotein ACA as a membrane receptor, and its activation by means of crosslinking with ACA specific antibodies or its natural ligand(s) has been described in J. Biol. Chem. 2002; 277: 40472-40478; WO 2004/014948 A2 and WO 2005/105983 A1, the disclosure of these documents is incorporated herein by reference.

It has now surprisingly been found that by activating the surface glycoprotein ACA, a process is induced (via PLCγ/PI3K/Akt/PTEN/mTor) that promotes self-renewal in human blood progenitor cells leading to the generation of self-renewing human early progenitor cells, HSCs, and side population (SP) cells. In addition, it has now surprisingly been found that promoting self-renewal in adult progenitor cells by activating ACA induces pluripotency in these cells that leads to generation of pluripotent stem cells capable of *in vitro* as well as *in vivo* differentiation in all three germ layers, i.e. ectodermal cells, mesodermal cells and endodermal cells.

Thus, the present invention relates to a process for producing at least one selected from the group consisting of ectodermal cells, mesodermal cells and endodermal cells, comprising the step of activating the surface protein ACA.

The present invention further relates to a process for obtaining and/or maintaining pluripotent stem cells, comprising the step of activating the surface protein ACA.

The present invention further relates to a process for producing haematopoietic stem cells, capable of differentiating into all cells which constitute human blood including T-cells, comprising the step of activating the surface protein ACA.

The present invention further relates to a process for producing side population cells, comprising the step of activating the surface protein ACA.

The present invention further relates to a process for producing mesenchymal stem cells, comprising the step of activating the surface protein ACA.

The present invention further relates to a method of de-differentiating peripheral blood cells by activating the surface protein ACA.

The present invention further relates to an activator of the surface protein ACA for use in regenerative medicine and/or transplantation medicine.

The advantages of activating the surface protein ACA for the generation of a sufficient number of HSC (CD34+) cells are manifold. There is no need for toxic regiment in treating the patients like in leukapheresis, a common practice in transplantation medicine, no need for selection of CD34 positive cells and collection of a large amount of cells prior to amplification with cytotoxic drugs; no immunological problems of graft-versus-host disease. The source of stem cells is strictly autologous, easily accessible practically infinite steady-state peripheral blood, ex vivo manipulation straightforward by adding a small amount of ACA-specific antibody or it natural ligand(s). Generation of donor's own T-cells as well as natural killer cells (NK) not provided by common methods is one of the most important advantages. Immature status of cells obtained by the activation of ACA greatly reduces the risk of GvHD, thus permitting the use of HSC obtained by the activation of ACA in allogeneic settings as well.

The present invention also relates to a process for obtaining and/or maintaining pluripotent stem cells and shows for the first time an efficient genetic modifications-free method for derivation of pluripotent stem cells.

Blood-derived pluripotent stem cells obtained by activating ACA bear the entire marker characteristics for human embryonic stem cells and can be differentiated as it is proven by *in vitro* as well as *in vivo* methods into cells of all three germ layers, represented by neural (ectoderm), hepatic (endoderm) or myocardial (mesoderm) cells. Furthermore, they do not form teratoma and therefore they are far more advantageous to all other methods available world-wide based on human embryonic stem cells or induced pluripotent stem cell (iPS) technologies. Moreover, ACA-generated stem cells, as it is the case for all somatic cells, do not bear the medical, ethical and legal problems associated with fetal or embryonic stem cells.

As a signaling molecule, ACA is involved in all developmentally conserved signaling pathway(s) known to shape the structure of embryo, but influencing somatic stem cell compartments indicating that the universal pathway(s) regulating all these processes must exist *in vivo.* The experiments illustrated below illuminate the ACA molecular mechanism which is highly likely to be responsible for replenishing the human body with stem cells having appropriate developmental potential needed in case of injury. Receptor occupancy of ACA through antibody crosslinking and activation of series of phosphatases and other mediators of ACA signaling mechanism imply that ACA activity must be regulated *in vivo* through small molecules, e.g. soluble natural ligand(s). Binding of natural ligand(s) to ACA receptor and initiation of ACA signaling network is likely to determine the level of de-differentiation and consequently the fate of stem cells, and can be exploited for the generation of all types of human stem cells from adult to pluripotent, without overgrowth of immature cell type.

Activating the ACA specific signaling network with its specific antibodies as well as its natural ligand(s) enables the generation of human stem cells capable of engrafting all mice organs in animal testing and therefore could be considered as the most relevant method which can be applied for adoptive cell therapy of damaged and/or degenerated tissues.

Hereinafter, details of the present invention and other features and advantages thereof will be described. However, the present invention is not limited to the following specific descriptions and embodiments, but they are rather for illustrative purposes only.

The expression "comprising" as used herein includes not only the meaning of "comprising" but also encompasses "consisting essentially of" and "consisting of".

The present invention relates to a process for producing at least one selected from the group consisting of ectodermal cells, mesodermal cells and endodermal cells, comprising the step of activating the surface protein ACA.

The term "surface protein ACA" as used herein denotes the natural glycosylphosphatidylinositol(GPI)-anchored glycoprotein, described in WO 2004/014948 A2, the disclosure of which is incorporated herein by reference, as well as a protein having an amino acid sequence differing from the wild-type sequence, such as by deletion(s), substitution(s) and/or addition(s) of amino acids, but which still exhibits a similar biological activity to the natural protein.

The term "activating the surface protein ACA" as used herein includes the activation of the protein itself and/or the activation of the expression of the protein.

The ectodermal cells produced by the process according to the present invention in particular comprise neuronal cells, epidermis cells, and progenitor cells thereof.

The mesodermal cells produced by the process according to the present invention in particular comprise smooth muscle cells, cardiac muscle cells, renal cells, skeletal muscle cells, bone cells, blood cells, in particular red blood cells, and progenitor cells thereof.

The endodermal cells produced by the process according to the present invention in particular comprise hepatic cells, pancreatic cells, enteric cells, respiratory tract cells, endocrine gland cells, urinary system cells, and progenitor cells thereof.

The present invention further relates to a process for obtaining and/or maintaining pluripotent stem cells, comprising the step of activating the surface protein ACA.

The term "pluripotent stem cells" as used herein denotes stem cells that can cross the lineage boundaries and can differentiate into cells of all three germ layers.

The term "maintaining pluripotent stem cells" as used herein denotes that the pluripotent stem cells substantially maintain their status of pluripotency, i.e. that a differentiation of the pluripotent stem cells to more specialised stem cells or specific cells is minimized or prevented.

The present invention further relates to a process for producing haematopoietic stem cells, capable of differentiating into all cells which constitute human blood including T-cells, comprising the step of activating the surface protein ACA.

The term "haematopoietic stem cells" as used herein denotes cells capable of unrestricted self-renewal as well as multilineage differentiation into progenitor of all mature blood cell lineages including T-cells.

The present invention further relates to a process for producing side population cells, comprising the step of activating the surface protein ACA.

The present invention further relates to a process for producing mesenchymal stem cells, comprising the step of activating the surface protein ACA.

In a preferred embodiment, these cells are produced or obtained from adult cells, in particular blood cells, preferably peripheral blood cells.

The term "adult cells" as used herein encompasses all sorts of non-embryonic cells, i.e. also encompasses juvenile cells.

The present invention further relates to a method of de-differentiating peripheral blood cells by activating the surface protein ACA.

The term "de-differentiating" as used herein denotes a regression of a specialized cell, such as peripheral blood or other adult cells, to a more primitive, more embryonic, unspecialized form.

The present invention is in particular based on the finding that by activating the surface protein ACA, peripheral blood cells can be de-differentiated to blood progenitor cells, further to haematopoietic stem cells, even further to side population cells, and finally even to pluripotent stem cells. These pluripotent stem cells are capable of differentiating into ectodermal cells, mesodermal cells and endodermal cells.

The activation of the surface protein ACA typically takes place via a sequence of PLCγ/PI3K/Akt/PTEN/mTOR.

In a further preferred embodiment, the surface protein ACA is activated by an antibody to ACA. An antibody to ACA can be obtained, as described in WO 2004/014948 A2, the disclosure of which is incorporated herein by reference.

In a particularly preferred embodiment, the step of activating the surface protein ACA comprises contacting blood cells, preferably peripheral blood cells, with an activator of the surface protein ACA, in particular with an antibody to ACA.

It is possible to carry out the processes and method according to the present invention *in vivo* as well as *in vitro.*

The present invention further relates to an activator of the surface protein ACA for use in regenerative medicine and/or transplantation medicine.

The term "regenerative medicine" as used herein denotes the process of replacing or regenerating cells, tissues or organs to restore or establish their normal function.

Activators of the surface protein ACA typically act as agonists at the ACA protein. In addition, such activators comprise molecules identified by the use of a recombinantly produced ACA protein, i.e. a recombinantly produced ACA protein can be used to screen for and identify activators, such as by exploiting the capability of potential activators to bind to the protein under appropriate conditions. For instance, the activators can be identified by preparing a test mixture wherein the activators candidate is incubated with the ACA protein under appropriate conditions that allow ACA to be in a native conformation. Such an *in vitro* test system can be established according to methods well known in the art. Activators can be identified, for instance, by first screening for either synthetic or naturally occurring molecules that bind to the recombinantly produced ACA protein and then, in a second step, by testing those selected molecules in cellular assays for activation of the ACA protein, as reflected by activation of at least one of the biological activities. Such screening for molecules that bind the ACA protein can be easily performed on a large scale, such as by screening candidate molecules from libraries of synthetic and/or natural molecules.

Examples of the activator of the surface protein ACA include an antibody to the surface protein ACA as well as natural, semi-synthetic or synthetic ligands to the surface protein ACA. A preferred activator of the surface protein ACA is an antibody, in particular a monoclonal antibody, to the surface protein ACA.

The term "antibody" as used herein encompasses pooled monoclonal antibodies with different epitopic specifities, as well as distinct monoclonal antibody preparations. The term "antibody" as used herein further includes intact antibody molecules as well as antibody fragments, such as Fv, Fab and F(ab')2 fragments, which are still capable of specifically binding to ACA. Moreover, the term "antibody" as used herein encompasses chimerical, single chain and humanized antibodies.

### Brief Description of the drawings

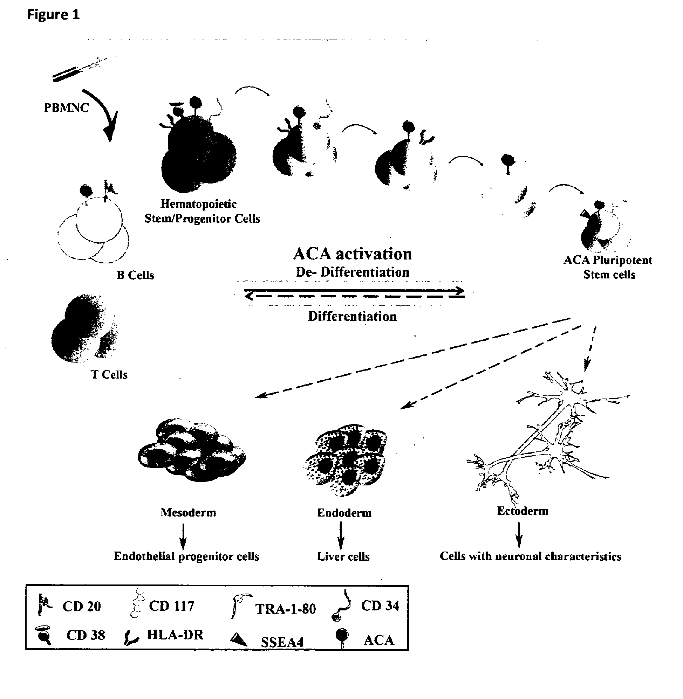

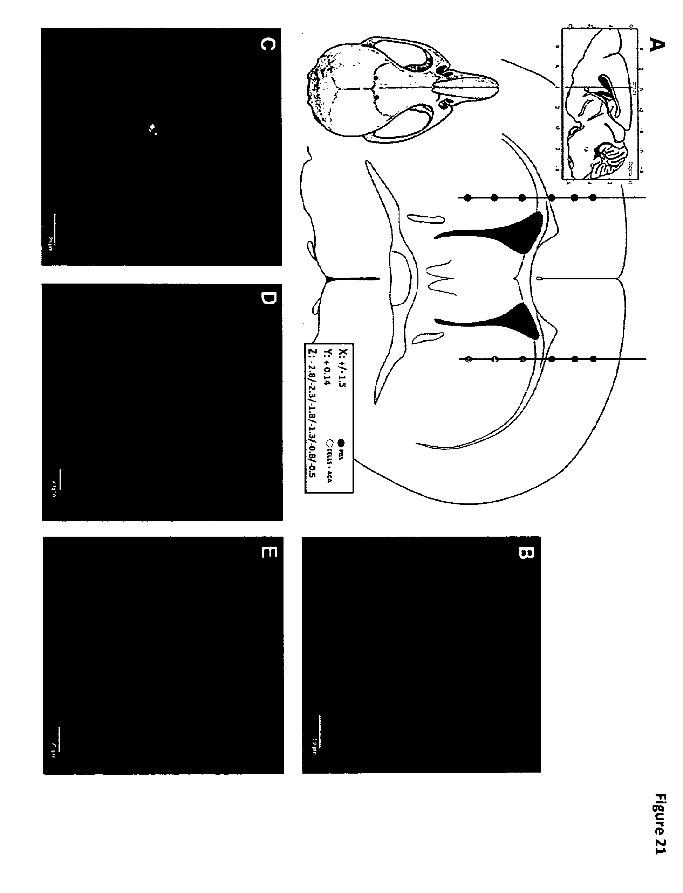

**Figure 1****: Activation by ACA is initiating sustained de-differentiation of human peripheral blood cells through mechanisms based on upregulation of Notch and WNT through PLCγ/PI3K/Akt pathway**
   Antibody crosslinking of ACA at the membrane of human peripheral blood mononuclear cells is leading to reprogramming of these cells, from haematopoietic progenitor over haematopoietic stem cells, side population cells over more primitive c-Kit cells until the cells finally reached the state of pluripotency, which is characterized by the expression of pluripotent stem cell-specific markers, like sialoprotein SSEA-4 and extracellular matrix proteins TRA-1-60, TRA-1-81. ACA pluripotent stem cells differentiate *in vitro* and *in vivo* into cell types of all three germ layers, ecto-, endo-, and mesodermal cell lines.

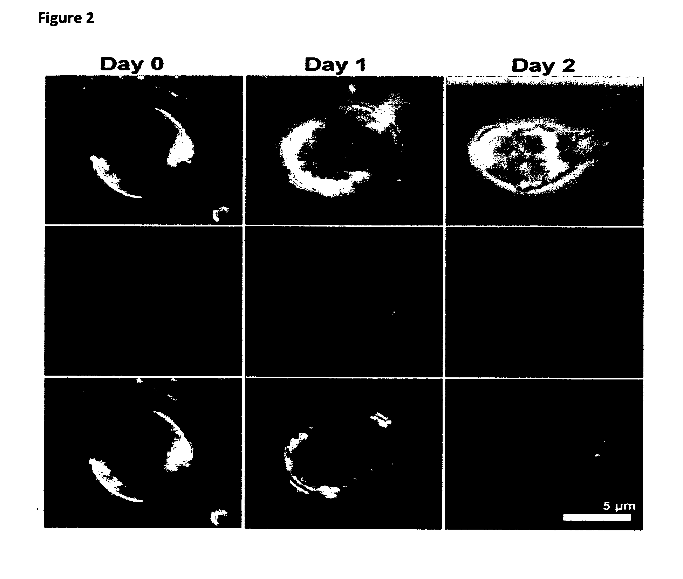

**Figure 2****: Antibody induced ligation of ACA protein on the cell membrane of peripheral blood mononuclear cells (PBMNCs) and lipid raft formation**
   PBMNCs were crosslinked with anti-ACA antibody, and cultured in appropriate medium. Images were acquired before and after crosslinking with ACA-specific antibody. Lipid rafts were stained with cholera toxin subunit B (CT-B) labeled with Alexa 555 fluorochrom and crosslinked with antibody specific to CT-B in human PBMNCs before, and 6h or 24h after activation by ACA with its specific antibody, followed by labeled secondary chicken anti-mouse Alexa 488. The formation of lipid rafts was visualized by spectral imaging confocal laser scanning system using a 60x magnification.

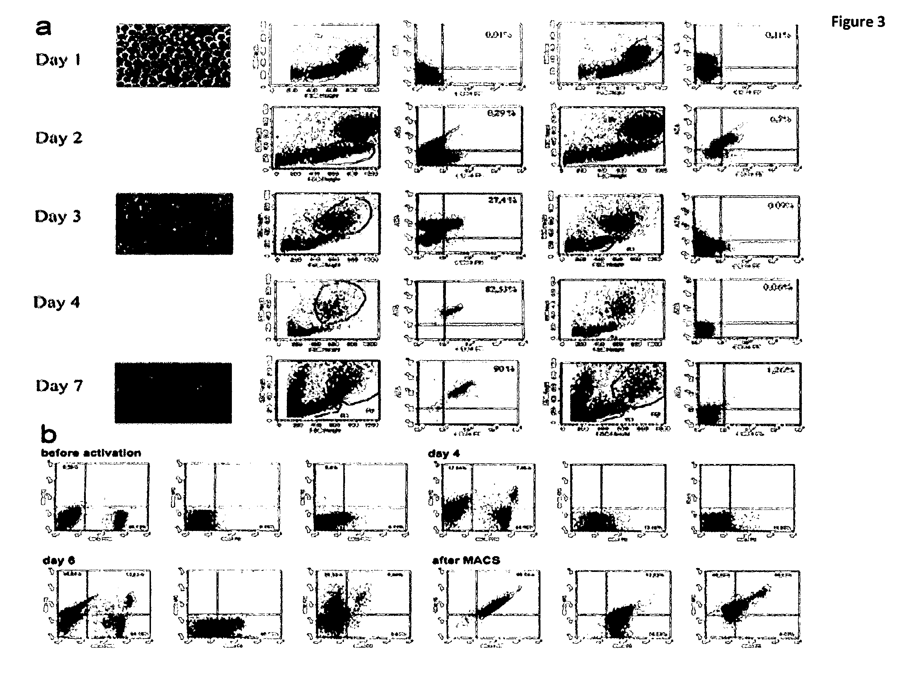

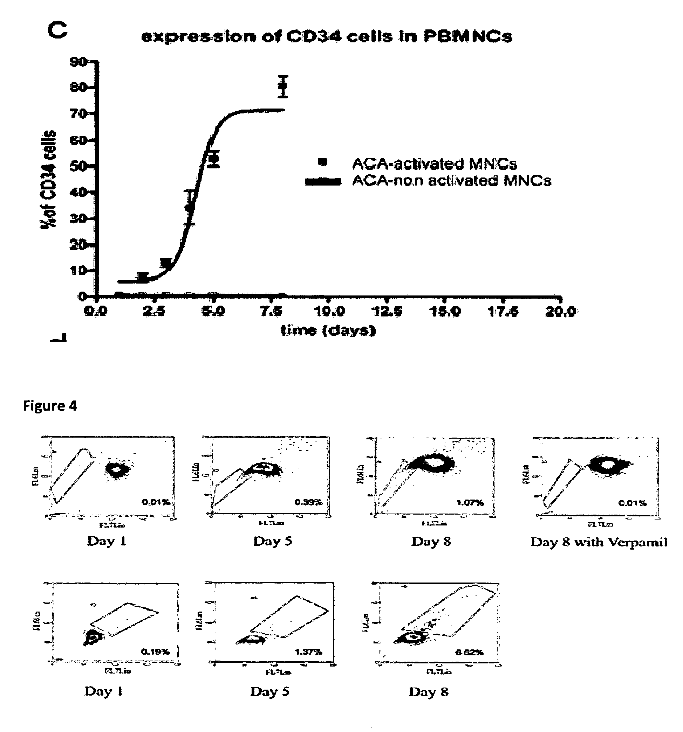

**Figure 3****: Generation of CD34 cells from PB**
   Mononuclear cells isolated from unmanipulated steady-state PB were crosslinked with ACA-specific antibody followed by Alexa 488 labeled chicken anti-mouse secondary antibody and phenotype of the new generated cells analyzed by means of fluorescence cytometry.
   a) A representative flow cytometry histogram shows the growing CD34⁺ cell population from day 1 to day 7. Percentage of CD34+ACA⁺ cells is shown by numbers. Immunophenotyping of newly generated cells stained with CD34 antibody is shown on the left.
   b) The numbers of CD38 expressing cells increases during 6 days culture time period upon ACA activation of PBMNCS, whereas the percentage of CD45 expressing cells decreases. Cell surface phenotype of immunomagnetically separated ACA-generated CD34 cells was analyzed by flow cytometry using CD34, CD133, CD90, and CD117 antibodies.
   c) Summary of data obtained from three different donors showing the generation of CD34⁺ cells by activation of ACA starting with unmanipulated PBMNCs. Error bars represent a standard error. As a negative control, PBMNCs from three various donors are cultured in Iscove's media supplemented with 10% FBS without ACA activation, and the number of CD34 cells plotted versus time (shown in red).
**Figure 4****: Generation of side population cells**
   Fluorescent activated cell sorting profile of ACA-generated human stem cells after staining with Hoechst 33342. The SP appears as the Hoechst low fraction capable of pumping out the dye. The SP cells are not present in PB, but they are generated after ACA activation, at day 5-8. SP cells are ablated when verapamil is included in the Hoechst incubation (upper panel). Lower panel shows the increase of proliferating cells after culturing of ACA cells upon ligation, as a function of time.

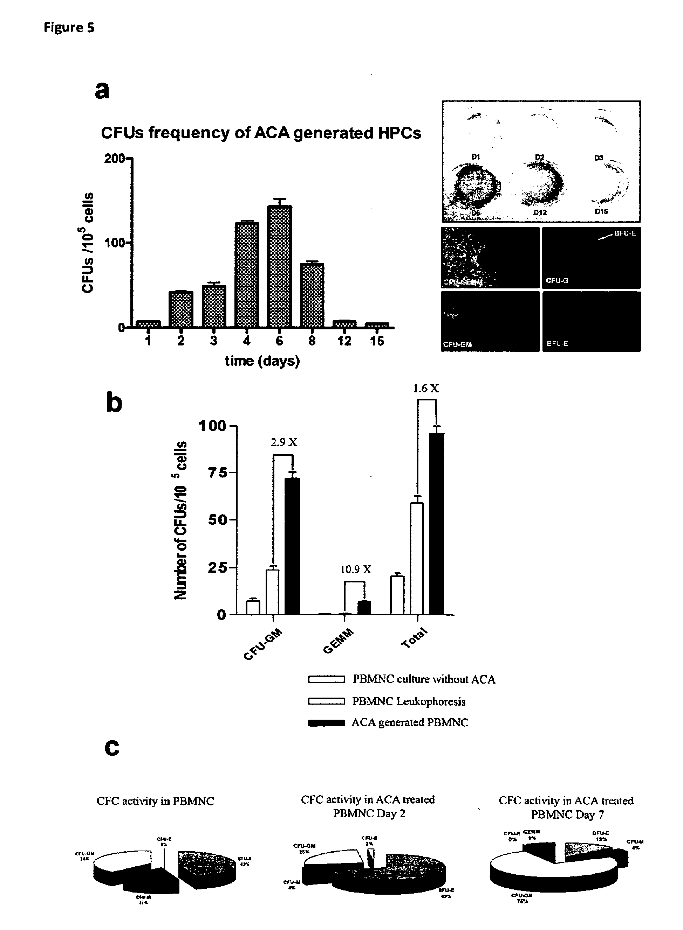

**Figure 5****: *In vitro* HPC assays of ACA-generated human progenitor cells**
   The capacity of ACA-generated progenitor cells to differentiate into various blood lineages in tissue culture was measured using methyl cellulose supplemented with various cytokines known to support haematopoietic differentiation.
   a) Total replating frequency (number of colonies) in ACA-generated progenitor cells were assayed in time course experiment. The data represent the mean of three independent experiments; each was done in triplicate manner. Error bars represent standard errors. Micrographs of CFC plates carried out with PBMNCs treated with anti-ACA in time course experiments, is shown on right (top). A high magnification of typical colonies formed *in vitro* after ACA treatment is shown on right (bottom).
   b) Comparison of *in vitro* CFC activity of ACA-generated cells versus stem cells obtained through leukapheresis in routine clinical settings.
   c) Comparison of the colony types obtained from ACA-generated progenitors cells through the course of culture time. Note the increased presence of more primitive mixed colonies CFU-GM and CFU-GEMM as well as the decreased number of more mature lineage committed BFU-E after a longer culturing time of ACA activated PBMNCs. The results represent a mean value of three independent experiments.

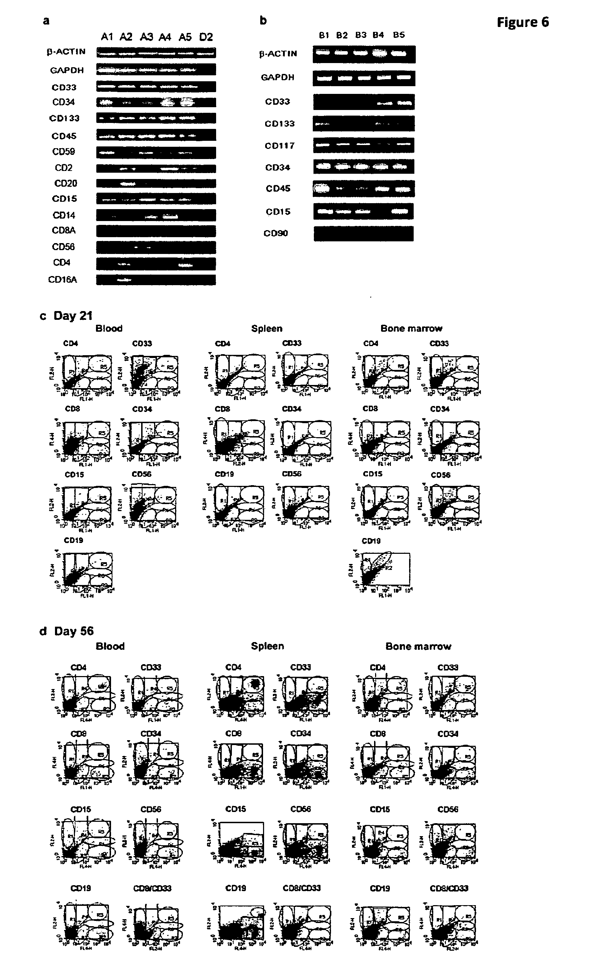

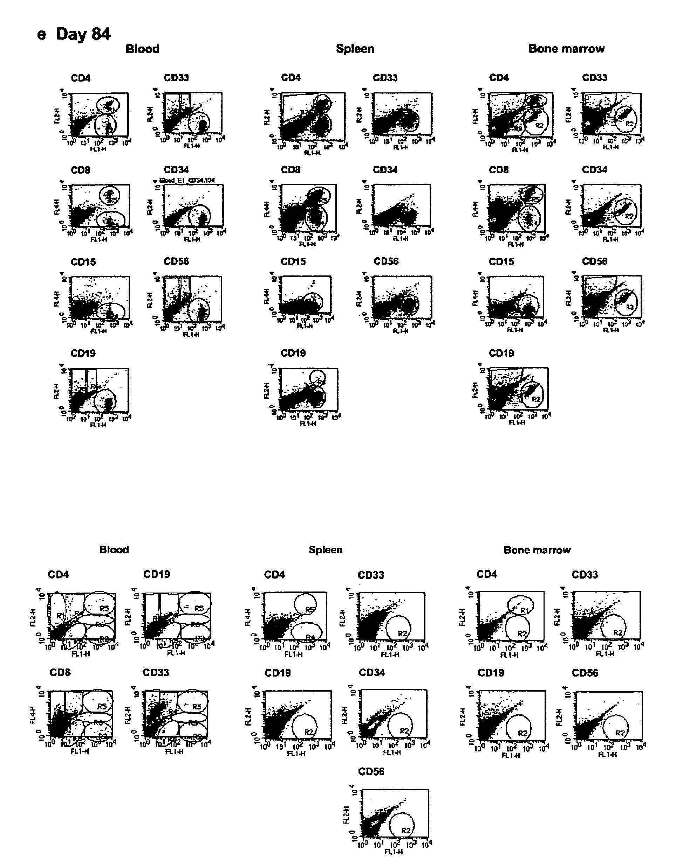

**Fig. 6****: *In vivo* assessment of ACA-generated haematopoietic stem cells**
   Functional property of ACA-generated stem cells was assessed after transplantation into sublethally irradiated host animals. Graded numbers of ACA-generated stem cells were injected; negative and positive controls received PBS and various phenotypes of ACA-generated stem cells, respectively. PCR analysis as well as flow cytometry of human lineage markers following transplantation were used to assess a SCID repopulation capacity of ACA-derived stem cells.
   a)PCR analysis of human engraftment of ACA-generated CD34⁺ cells
   b)PCR analysis of human engraftment of ACA-generated pluripotent-like stem cells.
      Culture of ACA-derived CD34 cells generates myeloid and lymphoid engraftment in NSG mice at day 21 or 56 post transplantation time.
   c) The mice were sacrificed at day 21 and bone marrow, blood and spleen cells were harvested for FACS analysis. Human myeloid engraftment was assessed by the percentage of cells positively stained with anti-human HLA antibody, CD15 and CD33. Lymphoid engraftment was assessed by the percentage of cells positively stained with CD19, CD4, and CD8. Human anti-CD56 antibody was used to estimate the population of NK cells.
   d) FACS analysis of human engraftment at day 56 post-transplantation time.
   e) Mice were sacrificed at day 84 post transplantation times and human engraftment was determined by flow cytometry using anti-human CD45 antibody alone or in combination with indicated antibodies to assess a differentiation potential of ACA cells.
   f) BM cells from primary transplanted NSG mice were injected into secondary mice and 10 weeks later human engraftment assessed by flow cytometry. Human cells were detected in all three haematopoietic organs mainly CD33, CD19, CD4, and CD8 confirming the long term engraftment potential of ACA generated HSCs.

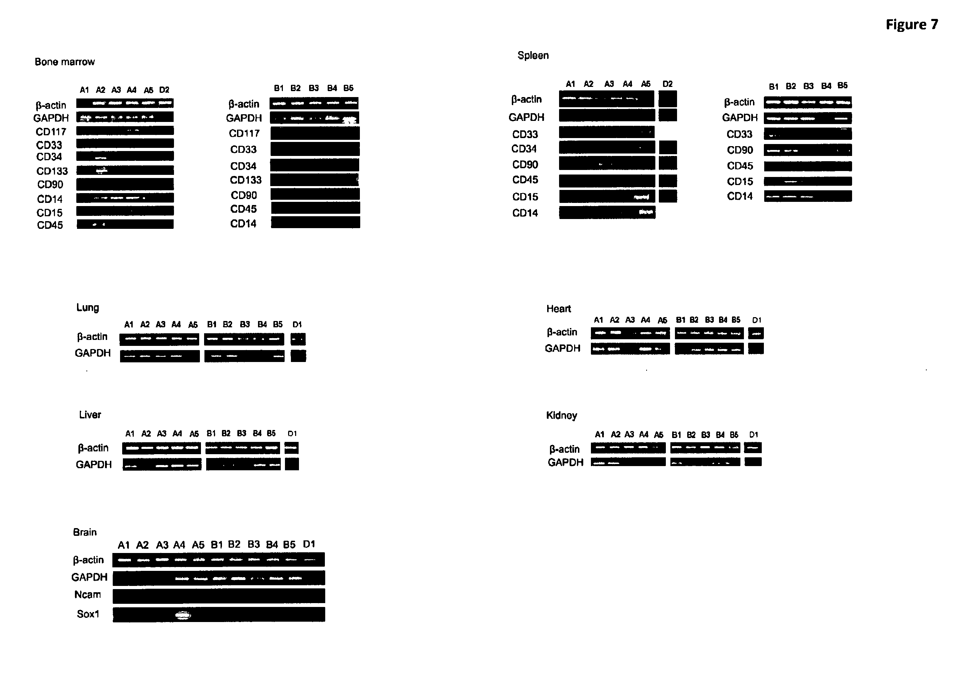

**Figure 7****: ACA-generated HSCs as well as pluripotent stem cells engrafted all organs in NSG mice**
   The analysis of involvement of human cells in various animal organs in xeno-transplantation assay was done by amplification of human-specific target gene (GAPDH) as well as genes characterizing more specific cells by means of RT-PCR analysis to specify a tissues specific differentiation potential of ACA-generated stem cells. Specific mouse β-actin primer was used to verify mouse cDNA. Group A animals received haematopoietic stem cells (CD34+), Group B pluripotent like stem cells.

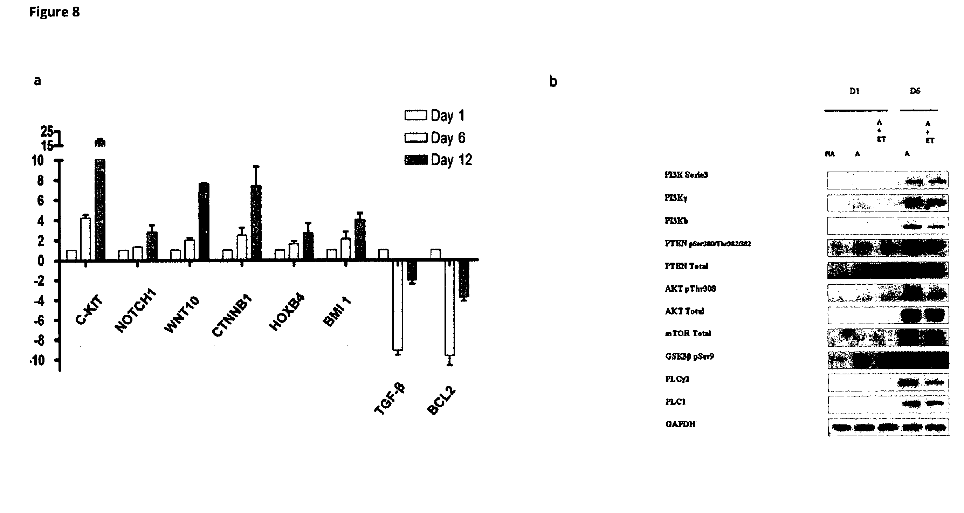

**Figure 8****: Regulation of gene expression after ACA-induced generation of stem cells and its specific inhibition with pharmacological inhibitors.**
   a) Live cells before (day 1), and after ACA activation at day 6 and 12 were isolated and total RNA of these cells was reverse transcribed and the expression of the indicated genes was analyzed by real-time analysis (TaqMan). Results were standardized by housekeeping gene GAPDH. Level of mRNAs was calculated by 2^{-ΔΔCT} method. Results were obtained from three independent experiments.
   b) ACA-induced activation of PI3K/Akt pathway is suppressed by PLCγ inhibitor ET-18-0-CH₃.
      PB ACA-generated haematopoietic stem/progenitor cells were cultured in Iscove's medium supplemented with 10% FBS in the absence or presence of inhibitor ET for 6 days before the preparation of total cell lysates, and the expression of phosphorylated PLC, PI3K, Akt, PTEN, mTOR, and GSK3ß was determined by western blot analysis. Non-activated mononuclear cells isolated from PB are used as controls. Blots were stripped and reprobed with GAPDH specific antibodies as a loading and transfer control.

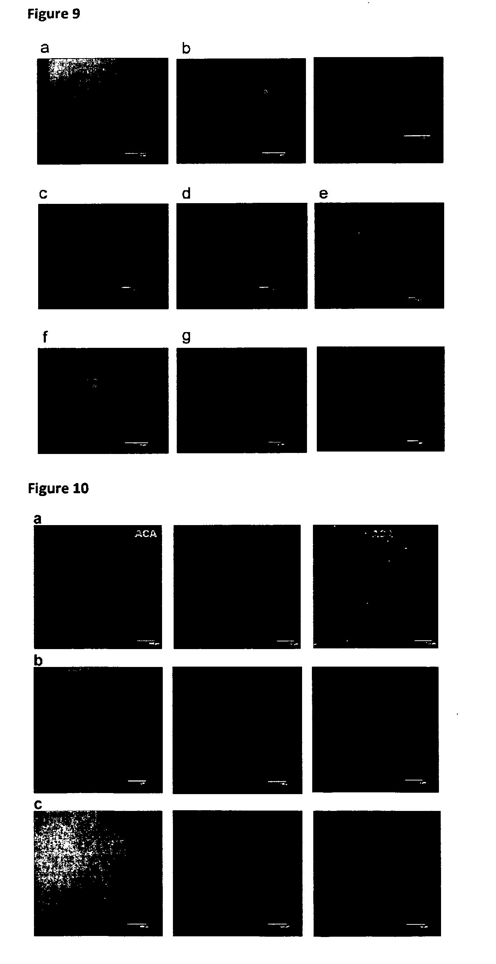

**Figure 9****: Oocyte and embryonic expression of ACA at the designated age**
   Human egg and embryo express a novel GPI-linked surface glycoprotein ACA.
   a) Human eggs were fixed with 4% pfa and mount with DAPI. The bright field picture was taken by confocal microscope.
   b) Fixed human egg was stained with ACA-specific antibody and mount with DAPI and the micrograph taken by confocal microscope.
   c-g) Post *in vitro* fertilized human embryo at day 1, 2, 3 and 5 (blastocyst) after fertilization were stained with ACA specific antibody versus DAPI, and ACA expression visualized by confocal microscopy.
**Figure 10****: Expression of ACA in hESC line H9**
   a) hESCs derived from H9 cell line grown on feeder cells were fixed with 4% of pfa and a colony of hESCs stained with antibody specific for ACA and ESCs marker TRA-1-81.
   b) Spontaneously differentiated ESCs were fixed and stained with anti-TRA-1-81 antibody. DAPI staining indicates the total cell content per field.
   c) Spontaneously differentiated ESCs upon ACA activation were stained with anti-TRA-1-81antibody. Staining with DAPI indicates the cell number per field.

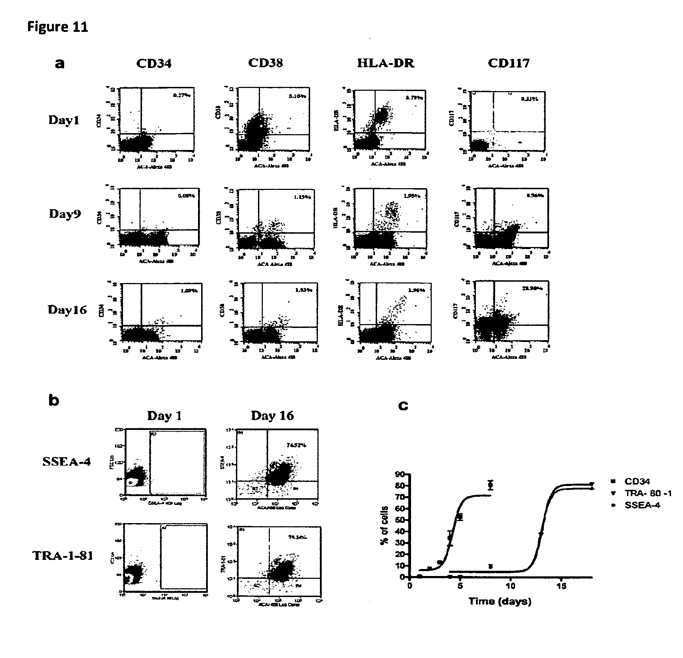

**Figure 11****: Peripheral blood derived pluripotent stem cells, generation and analysis**
   Mononuclear cells isolated from unmanipulated "steady state" peripheral blood were crosslinked with ACA-specific antibody and phenotype of new generated cells analyzed by means of fluorescence cytometry.
   a) A representative flow cytometry histogram shows the growing ACA/c-Kit population not present in PBMNCs, arising out of the cells which are losing their tissue specific markers due to cell reprogramming initiated by ACA.
   b) FACS analysis of human ESCs markers SSEA-4 and TRA-1-81 in pluripotent stem cells generated upon ACA activation.
   c) Summary of data obtained from various donors showing the generation of haematopoietic stem cells during the first week, and pluripotent stem cells after second week of culture time period upon activation by ACA.

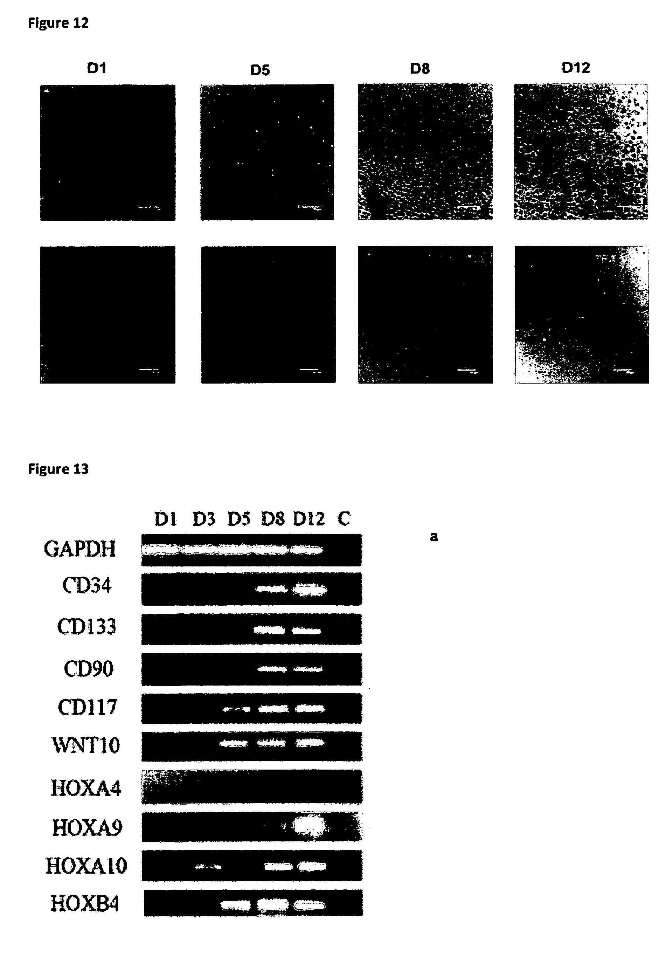

**Figure 12****: Confocal images of ACA-generated pluripotent stem cells**
   ACA activated PBMNCs were cultured in Iscove's media and analyzed for the expression of embryonic stem cell marker
   a) Blood-derived mononuclear cells upon ACA activation showing morphology changes from single cells at day 1 to small embryonic-stem cell like colonies with large nuclei and scant cytoplasm at day 12 during reprogramming procedure initiated by ACA. Micrographs are visualized by confocal microscopy.
   b) Human ACA stem cell colonies express markers common to pluripotent stem cells including SSEA-4, TRA-1-60, and TRA-1-81.

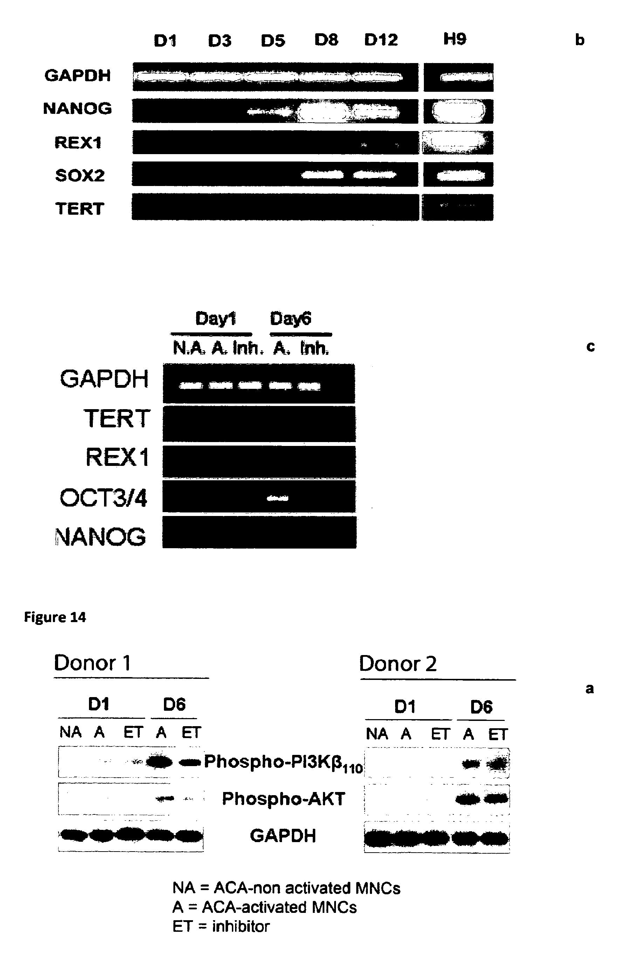

**Figure 13****: RT-PCR Analysis of ACA-generated haematopoietic stem cells and pluripotent stem cells**
   Semi-quantitative RT-PCR analysis of genes up-regulated upon activation by ACA was performed. The cells generated from PBMNCs upon ACA activation were taken at day 1, 3, 5, 8, and c-DNA was reverse transcribed from 2µg of total RNA, and gene-specific primers were used for amplification PCR. GAPDH was used as internal control.
   a) Peripheral blood ACA-generated haematopoietic stem cells
   b) Peripheral blood ACA-generated pluripotent stem cells expresses ES marker *NANOG, REX1, SOX2,* and *TERT.*
   c) RT-PCR analysis of pluripotency marker on ACA-generated cells in the presence or absence of PLC inhibitor ET.

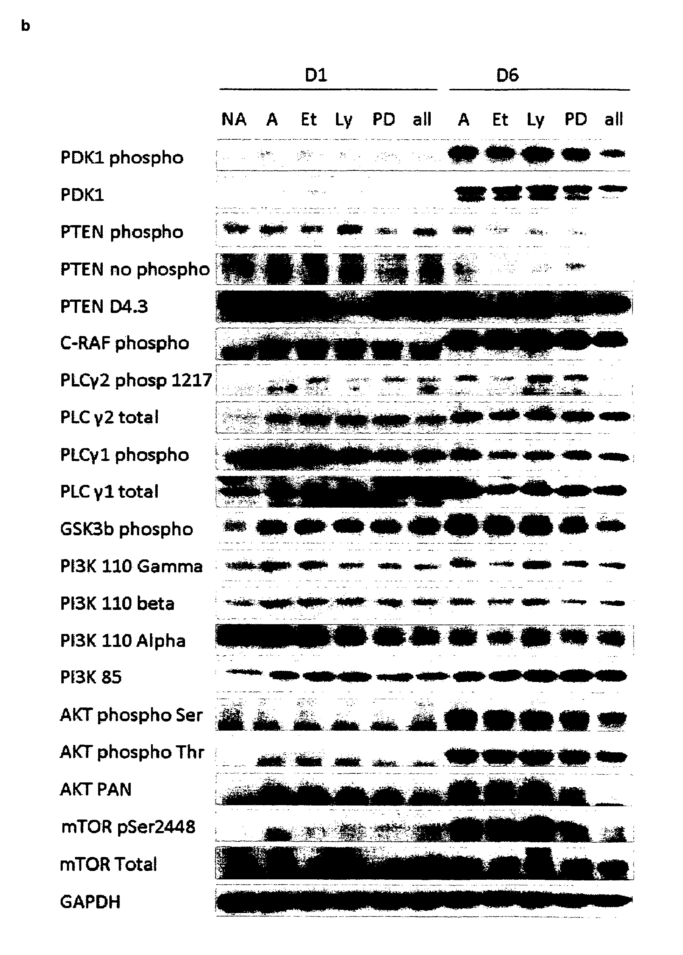

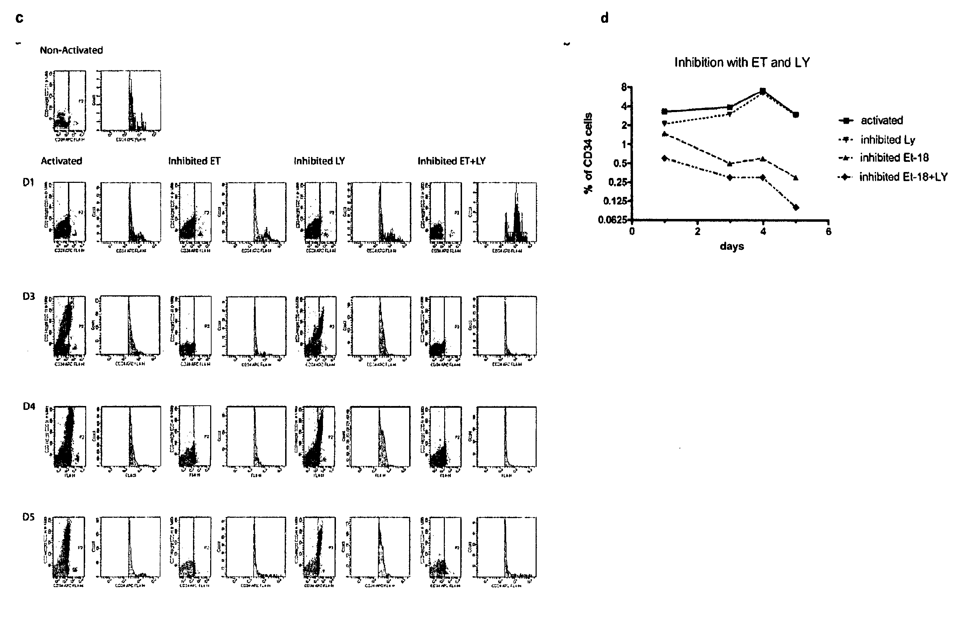

**Figure 14****: Relative protein expression and phosphorylation status of mediators of ACA signaling**
   Peripheral blood ACA-generated haematopoietic stem cells were cultured in the presence or absence of PLCγ inhibitor Et-18-0-CH₃ (ET) and cell-free extracts used to analyze the expression and phosphorylation status of indicated proteins by Western blotting. Non-activated mononuclear cells isolated from PB are used as control. Equal amount of protein was loaded for each sample. After SDS-PAGE, the proteins were transferred to the membrane and protein detected by corresponding phosho-specific antibodies. Lanes were loaded with 30µg protein and blots were striped and reprobed with antibodies to GAPDH as a loading control.
   a) Phosporylation pattern of PI3K and Akt of PBMNCs before and 6 days after ACA activation.
   b) Screening of the expression and phosphorylation status of proteins involved in ACA signaling mechanisms.
   c) ET and LY inhibited ACA-induced generation of CD34 cells. Flow cytometry analysis shows the generation of CD34+ cells during the presence or absence of ET, LY or ET+LY respectively in a 1-5 day culture time period.
   d) The total number of viable CD34+ cells in ACA cultures stimulated with, or without ET, LY or ET+LY was estimated by the flow cytometry. Presented are the data as a mean of +/-SEM for three independent experiments.

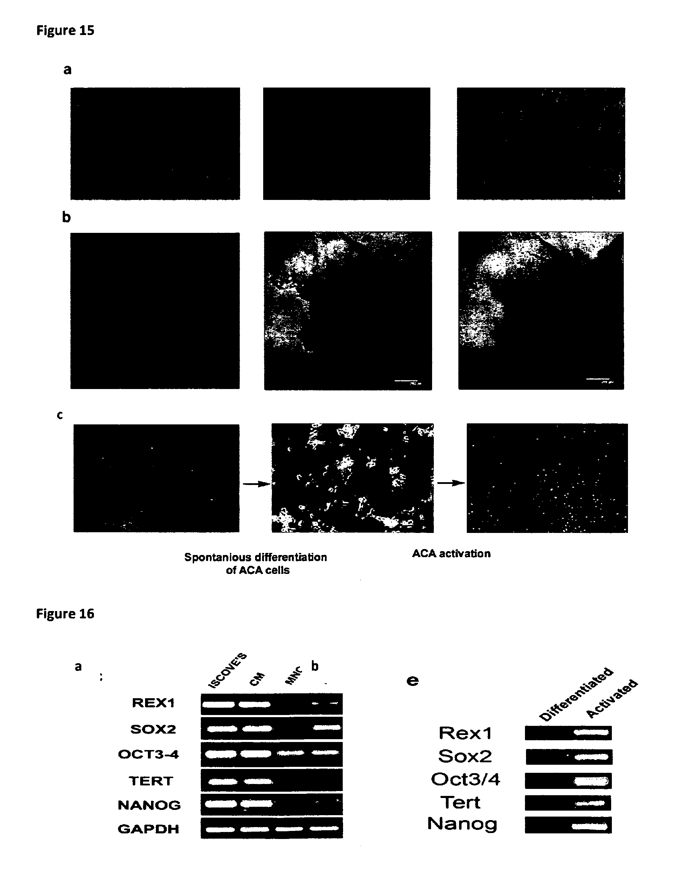

**Figure 15****: ACA-generated pluripotent stem cells are growing in suspension as well as on MEF layer**
   Blood derived mononuclear cells upon activation by ACA were grown in Iscove's medium supplemented with 10% FBS At day 8, part of the reprogrammed cells were harvested and plated onto MEF and continued to grow in MEF conditioned media. Large colonies were identified at day 16 of liquid culture, while the colonies of ACA cells growing on MEF appeared later.
   a) Morphology of ACA-generated pluripotent stem cells growing on MEF layer in conditioned medium. The micrographs were taken using phase contrast microscopy.
   b) Morphology of ACA-generated pluripotent stem cell colonies floating in liquid culture in Iscove's medium. Images were obtained using phase contrast and confocal microscopy.
   c) Phase contrast image of spontaneously differentiated colony of ACA-generated pluripotent stem cells before and after ACA treatment.
**Figure 16**: Semi-quantitative RT-PCR assay was used for expression analyses of ESCs marker genes in ACA-generated pluripotent stem cells.
   a) RT-PCR of ES marker genes in ES (H9), PBMNCs, and ACA-generated pluripotent stem cells grown in suspension in Iscove's medium, or on feeder layer in CM.
   b) RT-PCR analysis of pluripotent marker genes in spontaneously differentiated ACA stem cells colonies before and after activation by ACA.

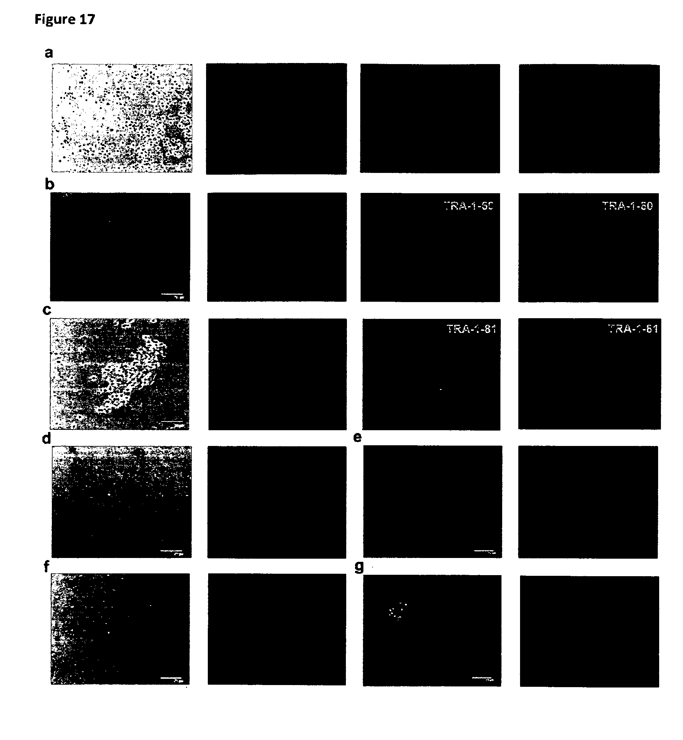

**Figure 17****: Immunohistochemistry of ACA cells expressing pluripotency markers SSEA-4, TRA-1-61, and TRA-1-81**
   ACA-generated pluripotent stem cells were grown on Matrigel coated plates in MEF condition media or in suspension and analyzed for expression of ES cell-specific markers.
   a-c) Immunostaining for pluripotency markers in ACA-generated stem cells growing on Matrigel coated plates. DAPI staining indicates the cell content per field. Images were taken by fluorescent microscopy.
   d-g) Immunostaining for pluripotency markers in ACA-generated stem cells grown in suspension. Confocal microscopic imaging was taken to analyze morphology and expression of pluripotency markers of ACA-generated cells.

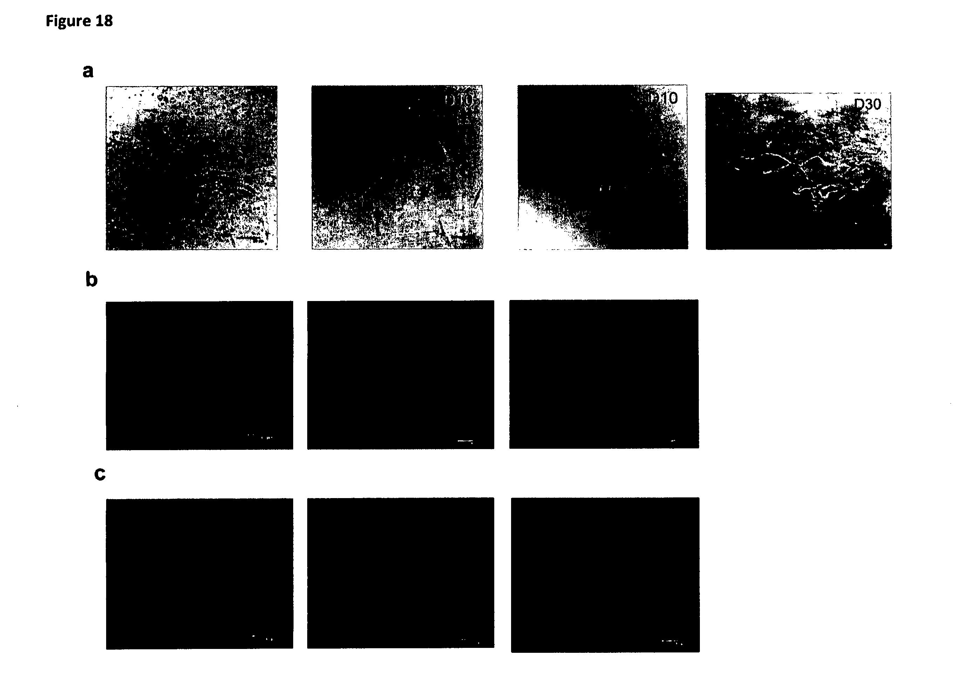

**Figure 18****: *In vitro* Differentiation of ACA-generated pluripotent stem cells.**
   ACA-generated pluripotent stem cells were subjected to lineage directed differentiation into ecto-, endo- and mesodermal lineages and analyzed by means of microscopic imaging, immuncytochemistry.
   a) Histological images of differentiated ACA cells growing in neuronal medium.
   b) Immunocytochemistry of ACA cells upon directed differentiation into hepatic cells showing expression α-feto protein, albumin and cytokeratin 18. Nuclei were stained with DAPI.
   c) Immunostaining of ACA cells upon directed differentiation into cardiomyocyte progenitors cells expressing mesodermal marker HCAM, VE-CAD and CD31.

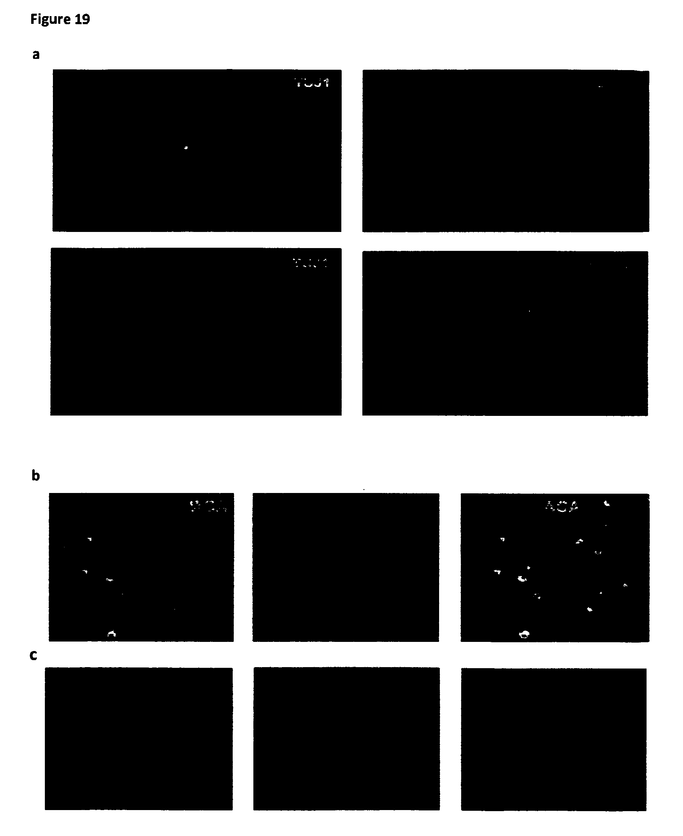

**Figure 19****: Immunophenotyping of ACA *in vitro* differentiated pluripotent stem cells**
   Targeted differentiation of ACA pluripotent stem cells in defined media generates ecto- and meso-dermal cells. ACA differentiated cells were analyzed for the presence of neuronal or endodermal cells using designated antibodies. Images were taken by fluorescent (Axiowert Zeiss) or confocal microscopy.
   a) ACA generated pluripotent stem cells grown in neuronal differentiation medium and the cells stained with human antibodies recognizing TUJ1, NESTIN and 04. DAPI was used for nuclear staining.
   b) ACA pluripotent stem cells grown in endothelial cell basal medium incubated with fluorescent labeled ac-LDL-Dil. Uptake was documented by fluorescent microscopy.
   c) ACA differentiated endothelial cells also express a vascular, endothelial growth factor KDR as documented using KDR antibody, a marker for cardyomyocytes progenitor cells.

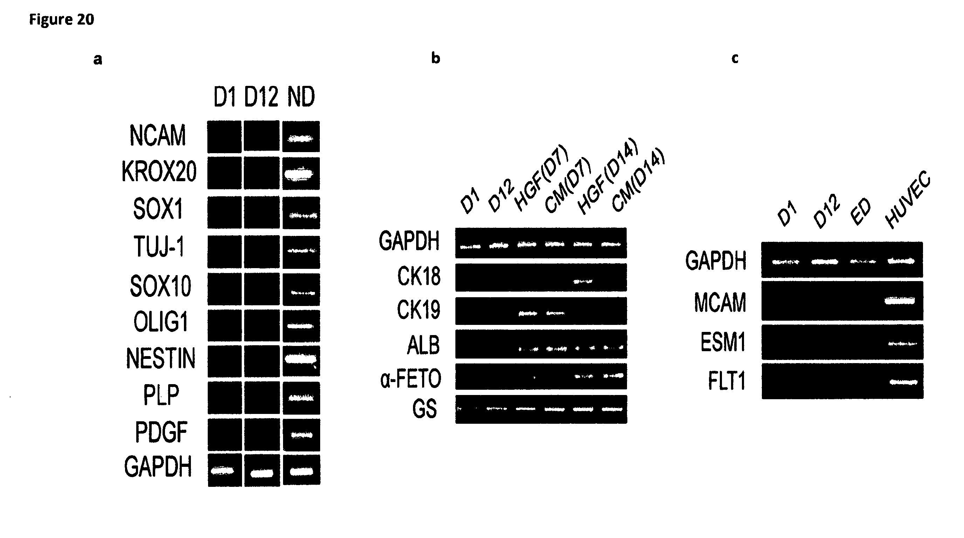

**Figure 20****: RT-PCR Analysis of ACA-differentiated pluripotent stem cells**
   RT-PCR analysis of various genetic markers, characterizing differentiation of ACA pluripotent stem cells into neuronal, hepatic and mesodermal cells.
   a) Neuronal
   b) Hepatic
   c) Mesodermal
**Figure 21****: ACA-generated pluripotent stem cells differentiate *in vivo* into neural precursor cells**
   Immunohistology of frozen section of engrafted mice transplanted with PKH 26 labeled ACA-generated pluripotent stem cells. DAPI was used to visualize nuclear DNA.
   a) A schematic overview of *in vivo* transplanted ACA-generated pluripotent stem cells
   b) Human nuclei antibody is used to characterize engrafted human cells.
   c) Transplanted cells express human GPI-linked glycoprotein ACA.
   d) ACA pluripotent stem cells differentiate into neural precursor cells expressing vimentin.
   e) Engrafted ACA pluripotent stem cells differentiate into neural cell marker N-CAM.

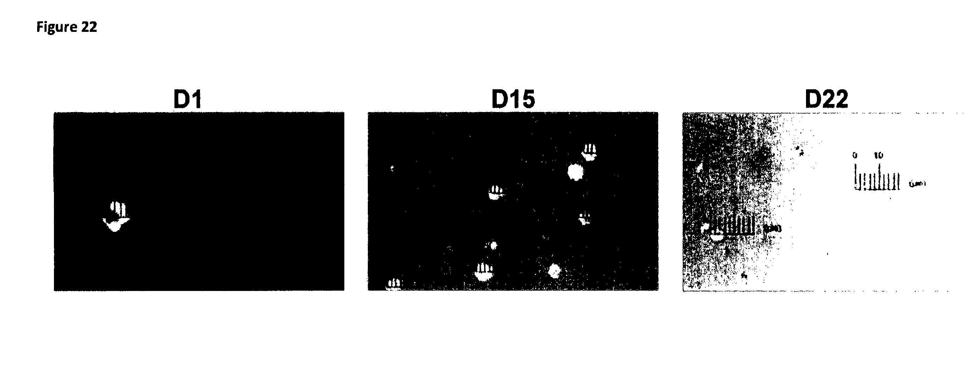

**Figure 22****: The cell size of *de novo* generated stem cells alongside the reprogramming by ACA**
   PBMNCs activated by ACA were cultured in an Iscove's medium supplemented with a 10% FCS, *de novo* ACA-generated cells were stained with ACA specific antibody and the size of cells was estimated throughout culture time.

### Experiments

### Generation and culturing of human stem cells

Human peripheral blood mononuclear cells (PBMNCs) were isolated by Lymphoprep (Axis Shield, UK), centrifuged and crosslinked with ACA-specific antibody, cultured and maintained in suspension in Iscove's modified Dulbecco's medium (IMDM) supplemented with 10% FBS (Gibco Life Techologies, Grand Island, NY). The cells were taken at different time points for the semi-quantitative RT-PCR, quantitative RT-PCR (real-time analysis), for immunophenotyping by flow cytometry and immune cytology and finally for *in vitro* or *in vivo* analysis of stem cell function.

For the generation of haematopoietic stem cells (HSC) mononuclear cells from PB were isolated and ACA activated as described, the new generated cells were grown for 4-7 days in IMDM medium supplemented with 10% FBS. Side population cells were generated at day 8-9; pluripotent stem cells were generated at day 10-14 using the culture conditions as described above.

For growing of ACA pluripotent stem cells on feeder cells, MEF(mouse embryonic media strain CF-1) mitotically inactivated by radiation were prepared according to standard protocol and approved by the ethics committee for animal care established at Principe Felipe Research Centre Valencia, Spain. At day 8 of ACA growing PBMNCs culture in Iscove's medium upon crosslinking with ACA antibody, cells were placed on freshly prepared MEF layer and further cultivated in ESC medium consisted of Knockout-DMEM (Invitrogen, Carlsbad CA), 100µM β-mercaptoethanol (Sigma, St Lois), 1mM L-glutamine (Invitrogen Carlsbad CA), 100mM nonessential amino acids, 20% serum replacement (SR; Invitrogen), 1% penicillin/streptomycin (Invitrogen) and 8ng/ml bFGF (Invitrogen).Feeder-free cultivation of ACA pluripotent stem cells was performed to assess the expression of hESCs antigens by means of immunofluorescence. For that purpose, ACA pluripotent stem cells were growing in MEF-condition media supplemented with 8ng/ml bFGF on culture dishes coated with Matrigel 1:30 (BD Bioscience, Franklin Lakes, NJ).

Recovery of the pluripotent phenotype of differentiated H9 cell line was done by crosslinking of these cells with ACA-specific antibody. Cells were maintained in IMDM supplemented with 10% FBS.

### Generation and analysis of SP cells

Cells (1x10⁶ cells/ml) generated from PB after ACA treatment as described above, were incubated at 37°C with 5µg of Hoechst 33342 (Sigma Aldrich) in DMEM supplemented with 2% FBS and 10mM Hepes for 90min and subsequently were subjected to flow cytometry-based analysis. Hoechst exclusion was inhibited by adding 50µg verapamil. Hoechst-DNA binding was detected by excitation with a UV laser (355nm at 50mW), followed by a 610nm shortpass dichroic mirror and 670 LP and 450 BP 20 detection filters for red and blue emission wavelengths, respectively.

### ACA-induced generation of stem cells and its specific inhibition with pharmacological inhibitors.

Mononuclear cells isolated from peripheral blood were cultured in Iscove's media supplemented with 10% FBS, pre-incubated with the PLC γ inhibitor Et-18-CH₃ (ET) at 50µM, and PI3K inhibitor LY294002 (LY) at 20µM or ET +LY (Calbiochem USA) for 1h, and after ACA activation in the presence of inhibitors cultured at 37°C, as specified. After labeling with CD34 APC, CD45 FITC (BD Pharmingen) and CD 14 PE (e Bioscience), generation of CD34 cells was daily assessed by multiple flow cytometry analysis. The non-viable cells were excluded through staining with propidium iodide (PI). Conjugated isotype - matched irrelevant antibodies were used as controls.

### Flow cytometry

The expression of activation or lineage associated antigens in ACA-generated stem cells was assessed by time course flow cytometry

MoFlo, FACS Calibur or FACS Cytomic FC 500 was used for analysis of cell subpopulations and sorting. Fluorescent phycoerythrin (PE) conjugated CD34, CD38, HLA-DR monoclonal antibodies purchased by Dako Cytomation, CD90 (Beckton Dickinson (BD) San Jose CA, USA), CD117 (R&D Systems Minneapolis, MN USA) were used for phenotypic analysis of haematopoietic stem/progenitor cells generated from PB upon activation of ACA. Gating was done with matched isotype control monoclonal antibodies.

*In vivo* labeling of lipid rafts was performed using Alexa Fluor 555 labeled CT-B (Invitrogen) followed with crosslinking using anti-CT-B and images taken by confocal microscopy. Newly ACA-generated HSCs, were seeded in 4 wells plate, and stained with CD34 antibody and images taken by confocal microscopy.

BM, spleen, and PB of NOD/SCID transplanted mice were analyzed for the presence of human haematopoietic cells at days 21 and 54 post-transplant time by flow cytometry using HLA-I-FITC, allophycocyanin (APC) labeled HLA-I, HLA-I-Alexa 488 ( Becton Dickinson) alone or in the combination with CD4 PE, CD15PE, CD19PE CD56PE, CD33PE, CD8 APC (Miltenyi, Biotec) antibodies. Conjugated isotype-matched irrelevant mAbs were used as controls.

In another experiment the engraftment of human cells generated at day 84 post-transplant time as well as post secondary transplant engraftment, were tested using human CD45 FITC, CD34PE, (Beckton Dickinson), CD34PE, CD4PE, CD15 APC, CD19PE, CD56PE, CD33PE, CD8APC (Miltenyi Biotec) antibodies. Fluorescence intensity of isotype controls as well as that of the differentiation markers measured for control animals were deducted from the value obtained for the expression of engrafted animals in all experiments.

TRA-1-81PE, TRA-1-61 PE and SSEA-4 Alexa 488 (e Bioscience, San Diego, USA) were used for phenotypic analysis of *de novo* ACA-generated pluripotent stem cells.

### Clonogenic progenitor assays

Human clonogenic progenitor assays were performed by plating fresh human PBMNCs before and after ACA treatment, as well as CD34⁺ cells collected through leukapheresis, at the concentration of 1x10⁵ cells into methylcellulose cocktail (MethoCult H4434, Stem Cell Technologies, Vancouver Canada) containing 50ng/mL recombinant human (rh) SCF, 10ng/mL GM-CSF, 10ng/mL rhIL-3, and 3units/mL human EPO.

Cultures were incubated in a humidified atmosphere at 37°C and 5% CO2. The cultures were assessed at day 14 to 18 for the presence of BFU-E, CFU-E, CFU-GM and CFU GEMM according to supplier's instructions.

After two weeks in culture, differential colony counts were scored *in situ* by light microscopy morphological characteristics using an inverted microscope. Only colonies with more than 50 cells were scored. Each data point was generated from three replicates.

### Semi-quantitative RT-PCR analysis

The expression level haematopoietic, pluripotent, or differentiation genes were assessed by semi quantitative PCR analysis. Total RNA from ACA-generated cells was extracted using RNA Isolation IKit (Zymo Research, Orange, CA). After DNase treatment cDNA was isolated using Taqman Reverse Transcription Reagents (Applied Biosystems) according to manufacturer's instruction. The cDNA concentrations were normalized in all samples based on RT-PCR of GAPDH gene.

### Quantitative Real-Time PCR (TaqMan)

The transcript levels for candidate genes, known to play a role in self-renewal of HSCs, were assessed by quantitative RT-PCR analysis of PBMNCs before and on day 6 and 12 upon activation by ACA. Total RNA obtained from activated /or non-activated PBMNCs was independently reverse transcribed using TaqMan Reverse Transcription Reagents (Applied Biosystems, CA, USA) following the manufacturer's instructions. The analysis was performed with the ABI PRISM 5700 Sequence Detection System instrument and software (Applied Biosystems, CA, USA).

### In Vivo Analysis of HSC Function

PBMNCs were generated by means of ligation of ACA with its specific antibody, and ACA-generated human haematopoietic stem cells or pluripotent like stem cells were grown for additional three days in Iscove's medium supplemented with 10% of FCS and a limited numbers of cells were transplanted by tail vein injection into sublethally irradiated NOD/SCID IL-2Rγ^{-/-} mice according to standard protocols established by EPO Berlin-Buch Germany. The mice were sacrificed 21, 54, 84 days after transplantation in accordance to local animal welfare protocol and cells from blood, BM and spleen, were collected for FACS analysis of human cell engraftment.

The mice were sacrificed 8 or 12 weeks after transplantation with CD34⁺ or pluripotent stem cells and engrafted cells from blood, BM, spleen, heart, lung, kidney, and brain were collected for analysis of human engraftment. PCR for the human-specific gene GAPDH was used to assess human cDNA. Additionally, human-specific primers to the various human antigens were used for detailed assessment of human cell engraftment.

ACA-generated HSCs were tested in secondary transplantation experiments using BM cells of positive animals and FACS analysis performed as described above.

### Western Immunobloting

Phosphorylated and total primary antibodies were purchased from Cell Signaling Technology. GAPDH antibody was obtained from Santa Cruz Biotechnology. Western blot analysis was performed using standard techniques. In brief, ACA-generated cells were lysed in Triplex buffer (50 mM Tris HCl pH8, 120 mM NaCl, 0,1% SDS, 1% Nonidet P-40, 0,54 % deoxycholate. Equal amount of total lysate, 30 µg proteins per sample were analyzed by 10% SDS-polyacrilamide gel electrophoresis, and after blotting, membranes were incubated overnight at 4° C with primary antibodies, followed by HRP-conjugated antibody. The targeted protein was revealed using Pierce enhanced chemiluminescence kit according to supplier's instruction. Protein preparations of non-treated PBMNCs were used as controls.

### In vitro differentiation of ACA pluripotent stem cells

For neuronal differentiation, ACA- generated stem cells at day 8-10 were placed in ornithine/laminine- coated plates (Invitrogen) and cultured in GRM medium according to the published method (Erceg.at al., 2008 PLoS One 3, e2122).

For hepatic differentiation ACA-generated stem cells at day 8-10 were grown onto gelatin coated plates (0.1% gelatin in PBS (Invitrogen) as described in Kakinuma et al. 2003 Stem Cells 21, 217-227, or in DMEM supplemented with hepatocyte growth factor (HGF, 10 ng/ml).

For mesodermal differentiation ACA cells were placed on fibronectin coated plates and cultured in endothelial cell basal medium (EBM-2 Basal medium Lonza CC-3156) supplemented with EGM-2 MV single aliquots (Lonza CC-4176) consisting of 5% FBS, vascular endothelial growth factor (VEGF), fibroblast growth factor-B, (FGF-B), human epidermal growth factor B (hEGF), insulin-like growth factor-1, (R3-IGF-1), gentamicin + amphotericin-B (GA-1000), hydrocortisone, and ascorbic acid. The cells were replated at day 4, and all assays were performed by using cells harvested on day 7 with PBS plus 5mM/L EDTA.

For generation of mesenchymal stem cells, ACA cell were grown in basal medium for human mesenchymal stem cells supplied by Stem Cell Technology Vancouver, Canada.

### In vivo differentiation of ACA pluripotent stem cells

ACA-generated pluripotent stem cells at day 20 and day 22 respectively were labeled with PKH26 Red Fluorescent Cell Linker Kit (Sigma) and ca 6x10⁵ cells were transplanted in anesthetized (1.5-2% Isoflurane) mice (NOD/LtSz-Scid IL2Rγnull) positioned under a stereotaxic apparatus (Stoelting). ACA cells were injected unilaterally in one point, corresponding to the cortex and striatum. The 100-120 nl volume were injected in each of the 6 different points of the dorsal axe at the following coordinates from Bregma (L: ± 1.5; A: +0.14; D: -2.8/-2.3/-1.8/-1.3/-0.8/-0.5), according to the compact mouse brain atlas (Paxinos and Franklin, 2004Netherlands: Elsevier Academic Press, compact 2nd ed. Amsterdam). Control sterile PBS solution was injected performing the same surgical procedure in the contra lateral brain hemisphere applying the same volume and stereotaxic coordinates. After surgical procedure, grafted mice were returned to their cages and analyzed 3 months later.

### Immunocytochemistry

ACA-specific monoclonal antibody at the dilution 1:1000 was used to analyze the expression of ACA in fixed human oocytes and 1, 2, 3, and 5 days post *in vitro* fertilized embryos obtained from University Hospital La Fe Valencia, Spain. Goat anti-mouse Alexa 488 conjugated antibody (Molecular Probe) was used for secondary labeling according to suppliers instruction.

Neuronal differentiated cells were tested for the expression of neuronal marker using antibodies recognizing, rabbit anti-nestin (Chemicon, Millipore 1:200), mouse anti-O-4 (Chemicon, Millipore 1:100) and mouse anti-beta III tubulin-Tuj1 (Abcam 1:300).

Hepatic differentiation was assessed at day 8-10 after starting the hepatic differentiation, using following primary antibodies: anti-human ALB (DakoCytomation 1:500), and anti-human AFP (DakoCytomation 1:400) and anti-human cytokeratin CK18 (DakoCytomation 1: 25) according to supplier's instruction.

Mesodermal cells lineages were firstly assessed at day 4 upon mesodermal differentiation for their ability to endocytised acetylated apoprotein (acLDL). ACA cells were incubated in the presence of 10mg/ml of acetylated low density lipoprotein, labeled with 1,1'-dioctadecyl - 3,3,3',3'-tetramethylindocarbocyanine perchlorate, DiI-Ac-LDL (Biomedical Technologies, Inc). After 4h of incubation at 37°C, washed cells were fixed in 2% paraformaldehyde and analyzed by fluorescent microscopy using rhodamine excitation/emission filters. Immune detection of markers characterizing endothelial phenotype was performed at day 7 after the endodermal differentiation has started. The antibodies used to assess mesodermal differentiation were mouse anti-human HCAM (Santa Cruz Techology 1:100), mouse anti-human VE-cadherin (Santa Cruz Technology 1:100), CD31 PE (Becton-Dickinson 1:10) and KDR (Santa Cruz 1:50)

Primary antibodies were typically applied for 1h, washed three times with PBS for 5min and incubated with appropriate Alexa-Fluor 488 or Alexa 568 (Molecular Probe) conjugated secondary antibodies for 30min. After washing three times with PBS the cells were mounted on slides with ProLong ® Gold antifade reagent containing DAPI (Invitrogen).

The micrographs were taken in confocal or in Zeiss Axiowert 200M fluorescence microscope (Oberkochen, Germany).

For immunohistochemistry analysis of engrafted mice, animals were sacrificed and perfunded 3 months after injection of the cells, and the brains placed in sucrose overnight. Tissues in O.C.T. were cut at 5 and 10 microns and frozen sections were washed with PBS at RT and the slides were boiled in citrate buffer pH 6 for 30min (only for anti-human nuclei antibody). After washing three times with PBS and blocking for 20min with 0.2% Triton-X-100 and 2% BSA slides were incubated overnight at 4°C with primary antibodies. Primary antibodies in blocking solution were applied as follow: anti-human nuclei (Chemicon 1:100), anti-ACA (1:500), anti-vimentin (Santa Cruz 1:200) and anti-NCAM (Chemicon 1:200). Anti-mouse Pacific blue and anti-mouse Alexa 488 (Invitrogen 1:1000) was used as secondary antibodies. After treatment for 5min in 0.1% Sudan black in 70% ethanol (only for anti-human nuclei antibody) cells were washed and mounted as described. Prolong Gold antifade reagent with DAPI (Invitrogen), was used to visualize nuclear DNA. Micrographs were taken under confocal microscope.

While the present invention has been described in detail by way of specific embodiments and examples, the invention is not limited thereto and various alterations or modifications are possible, without departing from the scope of the invention.

## Claims

1. A process for producing at least one selected from the group consisting of ectodermal cells, mesodermal cells and endodermal cells, comprising the step of
activating the surface protein ACA.

2. The process according to claim 1, wherein the ectodermal cells are selected from the group consisting of neuronal cells, epidermis cells, and progenitor cells thereof.

3. The process according to claim 1, wherein the mesodermal cells are selected from the group consisting of smooth muscle cells, cardiac muscle cells, renal cells, skeletal muscle cells, bone cells, blood cells, and progenitor cells thereof.

4. The process according to claim 1, wherein the endodermal cells are selected from the group consisting of hepatic cells, pancreatic cells, enteric cells, respiratory tract cells, endocrine gland cells, urinary system cells, and progenitor cells thereof.

5. A process for obtaining and/or maintaining pluripotent stem cells, comprising the step of
activating the surface protein ACA.

6. A process for producing haematopoietic stem cells, capable of differentiating into all cells which constitute human blood including T-cells, comprising the step of
activating the surface protein ACA.

7. A process for producing side population cells, comprising the step of
activating the surface protein ACA.

8. A process for producing mesenchymal stem cells, comprising the step of
activating the surface protein ACA.

9. The process according to any one of the preceding claims, wherein the cells are produced or obtained from adult cells, in particular blood cells, such as peripheral blood cells.

10. A method of de-differentiating peripheral blood cells by activating the surface protein ACA.

11. The process according to any one of claims 1 to 9 or the method according to claim 10, wherein the surface protein ACA is activated via PLCγ/PI3K/Akt/PTEN/mTOR.

12. The process according to any one of claims 1 to 9 or 11 or the method according to claims 10 or 11, wherein the surface protein ACA is activated by an antibody to ACA.

13. The process according to any one of claims 1 to 9, 11 or 12 or the method according to any one of claims 10 to 12, wherein the step of activating the surface protein ACA comprises contacting blood cells with an antibody to ACA.

14. The process according to any one of claims 1 to 9 or 11 to 13 or the method according to any one of claims 10 to 13, wherein the process or the method is in vitro.

15. An activator of the surface protein ACA for use in regenerative medicine and/or transplantation medicine, in particular wherein the activator is an antibody to the surface protein ACA.
